# EUROPÄISCHE PATENTANMELDUNG

(11) **EP 2 520 219 A1**
(43) Veröffentlichungstag der Anmeldung: **07.11.2012**
(21) Anmeldenummer: 12164885.1
(22) Anmeldetag: 20.04.2012
(51) Int. Cl.: A61B 1/24, A61B 1/233, A61B 3/12, F21V 21/40, A61B 1/227

(54) **Instrumentenhandgriff für eine medizinische Diagnostikleuchte, medizinische Diagnostikleuchte und medizinisches Diagnosegerät**

(30) Priorität: 05.05.2011 DE 202011050045 U
(71) Anmelder: Kirchner & Wilhelm GmbH + Co. KG, 71679 Asperg (DE)
(72) Erfinder: Kirchner-Gottschalk, Regina, 71706 Markgröningen (DE)
(74) Vertreter: Kohler Schmid Möbus

(57) **Zusammenfassung**

Die Erfindung betrifft einen Instrumentenhandgriff (12) für eine medizinische Diagnostikleuchte (10), an das ein Licht-Instrumentenkopf (14) anschließbar ist, wobei der Instrumentenhandgriff (12) zur Aufnahme einer Energiequelle vorgesehen ist und/oder der Instrumentenhandgriff (12) eine elektrische Leitung (18) aufweist, die mit einer externen Energiequelle koppelbar ist. Im Instrumentenhandgriff (12) ist eine manuell betätigbare Lichtregeleinrichtung (20) zur Lichtstärkeregelung einer dem Licht-Instrumentenkopf (14) zugeordneten Lichtquelle (16) angeordnet. Die manuell betätigbare Lichtregeleinrichtung (20) ist zumindest teilweise hinter einer seitlichen Wandöffnung (22) des Instrumentenhandgriffs (12) angeordnet, und durch ein mit dem Instrumentenhandgriff (12) materialschlüssig verbundenes Abdeckelement (30) dichtend verschlossen. Das Abdeckelement (30) weist einen manuell eindrückbaren Betätigungsabschnitt (32) mit haptisch und optisch voneinander unterscheidbaren Markierungen (34) auf, wobei die Markierungen (34) als Oberflächenvertiefungen und/oder als Oberflächenerhebungen des Abdeckelements (30) ausgebildet sind. Das Abdeckelement (30) ist dem Instrumentenhandgriff (12) angespritzt und/oder mit dem Instrumentenhandgriff (12) verschweißt oder mit dem Instrumentenhandgriff (12) verklebt. Die Erfindung betrifft weiterhin eine medizinische Diagnostikleuchte (10) mit einem Instrumentenhandgriff (12) und ein medizinisches Diagnosegerät mit einer Diagnostikleuchte (10).

## Beschreibung

Die Erfindung betrifft einen Instrumentenhandgriff für eine medizinische Diagnostikleuchte, eine medizinische Diagnostikleuchte und ein medizinisches Diagnosegerät.

Derartige Instrumentenhandgriffe werden bei medizinischen Diagnostikleuchten, wie beispielsweise Ophtalmoskopen, Otoskopen und/oder Dermatoskopen für die medizinische Untersuchung im Hals-Nasen-Ohrenbereich, der Augen bzw. der Haut, eingesetzt.

Die Instrumentenhandgriffe weisen nach einer am Markt gängigen Bauart an ihrem einen Ende eine Kupplungseinrichtung zum Ankuppeln eines Licht-Instrumentenkopfes auf. Der Licht-Instrumentenkopf kann dabei beispielsweise ein Otoskop- oder ein Ophtalmoskopkopf sein. Die Licht-Instrumentenköpfe weisen zumeist eine integrierte Lichtquelle auf oder werden über eine im Instrumentenhandgriff angeordnete Lichtquelle bzw. eine externe Lichtquelle mit Licht versorgt.

Zum Regeln einer Lichtstärke der dem Licht-Instrumentenkopf zugeordneten Lichtquelle weisen die Instrumentenhandgriffe in bekannter Weise eine manuell betätigbare Lichtregeleinrichtung auf. Die Lichtregeleinrichtung ist im Instrumentenhandgriff häufig hinter einer seitlichen Wandöffnung des Instrumentenhandgriffs angeordnet bzw. erstreckt sich durch diese nach außen, so dass die Lichtregeleinrichtung mit einem Finger oder Daumen einer Bedienperson betätigt werden kann.

Bei einem Einsatz des Instrumentenhandgriffs am Patienten besteht immer die Gefahr einer Verschmutzung des Instrumentenhandgriffs, insbesondere mit potenziell infektiösen Körperflüssigkeiten, wie beispielsweise Sekreten und/oder Blut.

Die am Markt verfügbaren Instrumentenhandgriffe lassen sich, insbesondere im Bereich ihrer seitlichen Wandöffnung, bzw. auch innen oftmals nur unzureichend oder nur mit einem ungebührend hohen Aufwand reinigen.

Die Aufgabe der Erfindung besteht darin, einen Instrumentenhandgriff für eine medizinische Diagnostikleuchte sowie eine medizinische Diagnostikleuchte mit einem Instrumentenhandgriff und ein medizinisches Diagnosegerät mit einer Diagnostikleuchte dahingehend zu verbessern, dass diese mit geringerem Aufwand zu reinigen sind.

Die den Instrumentenhandgriff betreffende Aufgabe ist durch ein Instrumentenhandgriff mit den Merkmalen des Schutzanspruchs 1 und die die medizinische Diagnostikleuchte betreffende Aufgabe durch eine medizinische Diagnostikleuchte mit den Merkmalen des Schutzanspruchs 5 gelöst. Das Diagnosegerät nach der Lehre der Erfindung weist die in Anspruch 6 angegebenen Merkmale auf.

Vorteilhafte Weiterbildungen der Erfindung sind Gegenstand von abhängigen Ansprüchen.

Durch das materialschlüssig mit dem Instrumentenhandgriff verbundene Abdeckelement kann ein Eindringen von Verunreinigungen, wie beispielsweise potenziell infektiöser Sekrete oder von Blut in den Instrumentenhandgriff, insbesondere im Bereich der seitlichen Wandöffnung des Instrumentenhandgriffs, insgesamt vermieden werden. Dadurch erübrigt sich eine ansonsten zumindest bedarfsweise erforderliche Innenreinigung des Instrumentenhandgriffs. Weiterhin kann der Instrumentenhandgriff insgesamt auf vereinfachte Weise mittels einer Sprüh- und/oder Wischreinigung bzw. -desinfektion gereinigt/desinfiziert werden, ohne dass die Regeleinrichtung bzw. andere in dem Instrumentenhandgriff angeordnete elektrische oder elektronische Bauteile dadurch Schaden nehmen. Damit wird einer möglichen Infektionsgefahr einer Bedienperson entgegengewirkt und die Anwendungssicherheit des Instrumentenhandgriffs insgesamt erhöht. Auch kann der Instrumentenhandgriff ggf. in einer Reinigungs- bzw. Desinfektionslösung eingelegt, d. h., vollständig in diesen untergetaucht werden, was insbesondere bei höhergradigen Verunreinigungen bzw. einer erhöhten Kontaminationsgefahr mit Krankheitserregern von Vorteil ist. Das Abdeckelement ist dem Instrumentenhandgriff erfindungsgemäß angespritzt und alternativ oder zusätzlich mit dem Instrumentenhandgriff verschweißt oder aber mit dem Instrumentenhandgriff verklebt. Im erstgenannten Fall wird eine besonders kostengünstige Herstellung des Instrumentenhandgriffs erreicht. Darüber hinaus kann durch das Verschweißen des Abdeckelements mit dem Instrumentenhandgriff ein besonders hoher Abdichtungsgrad der seitlichen Wandöffnung des Instrumentenhandgriffs gegenüber einem Eindringen von Partikeln bzw. Flüssigkeiten erreicht werden. Zudem kann dadurch auf den Einsatz von Klebstoffen verzichtet werden, was im Hinblick auf ein späteres sortenreines Recycling von Vorteil ist.

Durch das erfindungsgemäße Abdeckelement kann zugleich der Bedienkomfort des Instrumentenhandgriffs erhöht werden, zumal das Abdeckelement einen verbesserten Grip, d. h., eine verbesserte Rutschfestigkeit, bietet, als dies bei im Stand der Technik bekannten Regeleinrichtungen mit zum Teil scharfkantigen Schiebe- oder Tastschaltern der Fall ist. Das Abdeckelement ermöglicht zudem ein besonders sicheres Halten des Instrumentenhandgriffs bzw. eines medizinischen Diagnostikleuchte mit einem solchen Instrumentenhandgriff in einer Halterung, beispielsweise eines medizinischen Diagnostikgeräts.

Darüber hinaus können Vorsprünge am Instrumentenhandgriff vermieden werden und die Oberfläche des Instrumentenhandgriffs kann über dessen gesamte Zirkumferenz eben gehalten werden.

Das Abdeckelement weist einen manuell eindrückbaren, d. h., in Richtung auf eine Längsachse des Instrumentenhandgriffs verstellbaren bzw. verformbaren, Betätigungsabschnitt auf. Dadurch können insbesondere Lichttaster der Lichtregeleinrichtung bequem und ohne großen Kraftaufwand betätigt werden. Die funktionelle Belegung der Lichttaster kann schaltungstechnisch in Abhängigkeit von einem jeweiligen Einsatzzweck des Instrumentenhandgriffs unterschiedlich gewählt sein.

Der Betätigungsabschnitt weist nach der Erfindung haptisch und optisch voneinander unterscheidbare Markierungen auf. Dadurch können Bedienfunktionen der von dem Abdeckelement überdeckten Regeleinrichtung sicher, schnell und fehlerfrei erfasst werden. Insgesamt wird dadurch die Bedienung des Instrumentenhandgriffs erleichtert. Die Markierungen sind als Oberflächenvertiefungen und/oder Erhebungen des Betätigungsabschnitts ausgeführt. Dies ist insbesondere bei einem behandschuhten Gebrauch des Instrumentenhandgriffs von Vorteil. Auch sind solche Markierungen gegenüber einem mechanischen Verschleiß bzw. einem Angriff von Reinigungs- bzw. Desinfektionsmitteln wenig empfindlich.

Zum Versorgen einer im Instrumentenhandgriff oder in einem an den Instrumentenhandgriff koppelbaren Lichtinstrumentenkopf angeordneten Lichtquelle mit elektrischer Betriebsenergie ist der Instrumentenhandgriff erfindungsgemäß zur Aufnahme einer Energiequelle, wie beispielsweise eines Akkumulators oder einer Batterie vorgesehen. Alternativ oder zusätzlich kann der Instrumentenhandgriff auch mit einer zum Anschluss an eine externe Energiequelle vorgesehenen elektrischen Leitung verkoppelbar bzw. verkoppelt sein.

Eine besonders robuste Bauart des erfindungsgemäßen Instrumentenhandgriffs zeichnet sich dadurch aus, dass das Abdeckelement den Instrumentenhandgriff vollumfänglich umgreift. Das Abdeckelement umgreift somit den Instrumentenhandgriff in dem im Betrieb des Instrumentenhandgriffs mechanisch besonders beanspruchten Umfangsabschnitt des Instrumentenhandgriffs manschettenartig. Ein unerwünschtes Lösen des Abdeckelements vom Instrumentenhandgriff wird dadurch sicher vermieden.

Nach einer bevorzugten Weiterbildung der Erfindung ist der Betätigungsabschnitt des Abdeckelements durch eine den Betätigungsabschnitt ringförmig umgreifende, vorzugsweise durchgehende, Nut von einem den Betätigungsbereich umgebenden Oberflächenbereich des Abdeckelements abgegrenzt. Dadurch kann der Betätigungsabschnitt auf vereinfachte Weise (blind) mit dem Bedienfinger bzw. Daumen aufgefunden werden.

Der Betätigungsabschnitt kann in diesem Zusammenhang zusätzlich oder alternativ auch nach innen in Richtung auf die Längsachse des Instrumentenhandgriffs gewölbt oder auch nach außen gewölbt sein (konkav bzw. Konvex).

Das Abdeckelement besteht vorzugsweise aus einem, vorzugsweise synthetischen, elastischen Polymer. Dadurch ergibt sich dadurch ein besonders sicheres Halten des Instrumentenhandgriffs. Zudem kann dadurch eine besonders hohe elektrische Isolation der Regeleinrichtung bzw. anderer stromführender Bauteile innerhalb des Instrumentenhandgriffs gegenüber der Umgebung erreicht werden. Dies bietet im Hinblick auf die Betriebssicherheit des Instrumentenhandgriffs Vorteile.

Nachstehend werden Ausführungsbeispiele der Erfindung anhand der Zeichnung näher beschrieben.

In dieser zeigt:
- Figur 1:: ein erfindungsgemäßes Ophtalmoskop für die medizinische Augendiagnostik, das einen erfindungsgemäßen Instrumentenhandgriff aufweist, in Vorderansicht;
- Figur 2:: ein Otoskop mit einem dem Instrumentenhandgriff aus Fig. 1 entsprechenden Instrumentenhandgriff, in Seitenansicht;
- Figur 3:: ein Otoskop mit einem Instrumentenhandgriff gemäß Fig. 1, in Seitenansicht; und
- Figur 4:: ein Otoskopie- und Ophtalmoskopiesystem mit dem Ophtalmoskop gemäß Fig. 1 und dem Otoskop gemäß Fig. 2, in Vorderansicht.

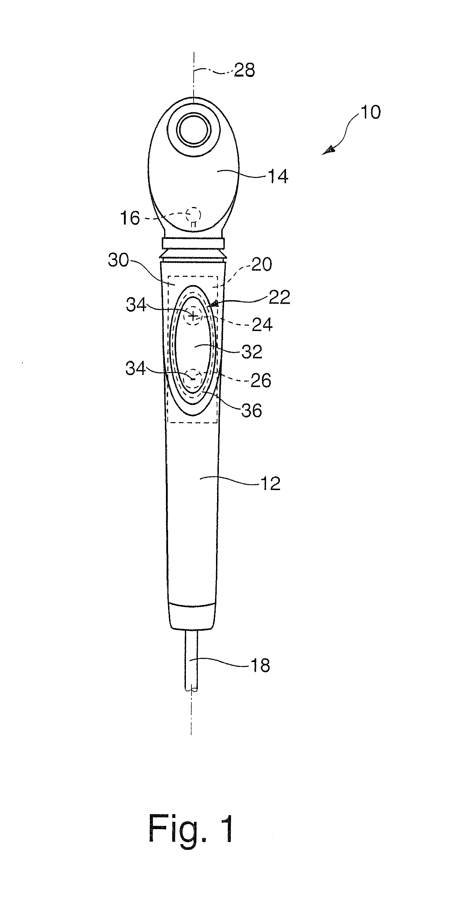

**Fig. 1** zeigt ein als Ophtalmoskop ausgebildetes medizinische Diagnostikleuchte **10** für die medizinische Augendiagnostik. Die Diagnostikleuchte 10 weist einen Instrumentengriff **12** auf, an das einenends ein Licht-Instrumentenkopf **14**, hier ein Ophtalmoskopiekopf, lösbar angeschlossen ist.

Der Licht-Instrumentenkopf 14 weist eine elektrisch betriebene Lichtquelle **16**, hier eine Xenon-Halogenlampe oder eine LED, zu deren Versorgung mit elektrischer Betriebsenergie ein an eine externe Energiequelle anschließbares elektrische Leitung **18** vorgesehen ist, die anderenends aus dem Instrumentenhandgriff 12 herausgeführt ist.

Die Lichtquelle 16 kann nach einer hier nicht näher gezeigten Ausführungsform der Erfindung auch innerhalb des Instrumentenhandgriffs 12 bzw. als eine mit dem Instrumentenhandgriff 12 koppelbare externe Lichtquelle 16 ausgebildet sein.

Zum Regeln der Lichtstärke des von dem Licht-Instrumentenkopf 14', d.h. dessen Lichtquelle 16, abzugebenden Lichts ist eine mit der elektrischen Leitung 18 sowie der Lichtquelle 16 verbundene Lichtregeleinrichtung **20** vorgesehen.

Die Lichtregeleinrichtung 20 ist im Instrumentenhandgriff 12 teilweise hinter einer seitlichen Wandöffnung **22** des Instrumentenhandgriffs 12, angeordnet und weist zwei Lichttaster **24**, **26** auf, die über die Wandöffnung 22 des Instrumentenhandgriffs 12 von außen manuell betätigbar sind.

Die beiden Lichttaster 24, 26 der Lichtregeleinrichtung 20 sind längs einer Längsachse **28** des Instrumentenhandgriffs 12 voneinander beabstandet angeordnet. Der in der Figur obere Lichttaster 24 dient einem Anschalten der Lichtquelle 16 sowie einem Hochregeln der Lichtstärke des von der Lichtquelle 16 abgegebenen Lichts, während der in der Figur untere Lichttaster 26 einem Herunterregeln bzw. einem vollständigen Ausschalten der Lichtquelle 16 dient.

Zum Schutz des Instrumentenhandgriffs 12 vor einem Eindringen von Verunreinigungen, wie beispielsweise Partikeln und/oder Flüssigkeiten (Sekrete, Blut etc.) ist die seitliche Wandöffnung 22 mittels eines Abdeckelements **30** dichtend verschlossen. Das Abdeckelement 30 weist einen Betätigungsabschnitt **32** auf, der den beiden Lichttastern 24, 26 unmittelbar aufliegt und diese von außen überdeckt. Der Betätigungsabschnitt 32 des Abdeckelements 30 ist zwecks einer Betätigung der Lichttaster 24, 26 in Richtung auf die Längsachse 28 des Instrumentenhandgriffs 12 soweit gegenüber der Längsachse 28 eindrückbar, dass die Lichttaster 24, 26 manuell betätigt werden können.

Der Betätigungsabschnitt 32 weist haptisch und auch visuell unterscheidbare Markierungen **34** auf, um die Funktion der unter dem Betätigungsabschnitt 32 angeordneten Lichttaster 24, 26 zu kennzeichnen. Die Markierungen 34 sind jeweils als Vertiefungen, d. h., als Oberflächenausnehmungen, des Betätigungsabschnitts 32 ausgeführt.

Der Betätigungsabschnitt 32 ist durch eine in diesem Ausführungsbeispiel oval ausgeführte Nut **36** von einem den Betätigungsabschnitt 32 umgebenden Oberflächenbereich **38** des Abdeckelements 28 abgegrenzt. Die Nut 36 erleichtert ein manuelles Auffinden des Betätigungsabschnitts 32 am Instrumentenhandgriff 12 mit einem Bedienfinger bzw. mit dem Daumen. Zudem dient die Nut 36 einer effektiven (elastischen) Rückführung des zum Betätigen der Lichtregeleinrichtung 20 eingedrückten Betätigungsabschnitts 32 des Abdeckelements 30 in dessen hier gezeigte Ruheposition.

Das Abdeckelement 30 ist dem Instrumentenhandgriff 12 formschlüssig angeformt und umgreift dieses im Sinne einer Manschette vollständig. Das Abdeckelement 30 ist dabei dem Instrumentenhandgriff 12 angespritzt und aus einem Elastomer, d.h. aus einem elastischen Polymer, gefertigt.

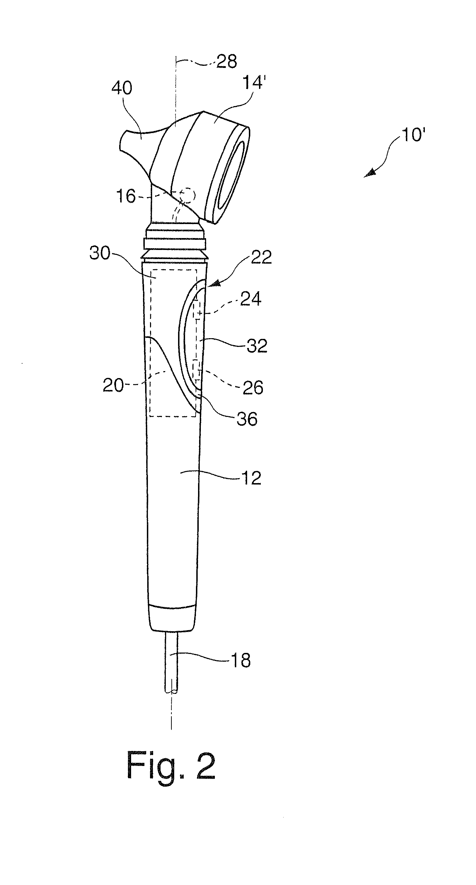

**Fig. 2** zeigt ein als Otoskop ausgebildetes medizinische Diagnostikleuchte 10'mit einem vorstehend erläuterten Instrumentenhandgriff 12. Im Gegensatz zu dem als Ophtalmoskop-Kopf ausgebildeten Licht-Instrumentenkopf 14 gemäß Fig. 1 weist das Otoskop einen als Otoskopkopf ausgebildeten Licht-Instrumentenkopf 14'auf, der in bekannter Weise mit einem Anschlusskonus **40** für einen nicht näher dargestellten Ohrtrichter versehen ist. In der Figur ist das den Instrumentenhandgriff 12 umfangsseitig vollständig umgreifende (manschettenartige) Abdeckelement 30 gut zu erkennen.

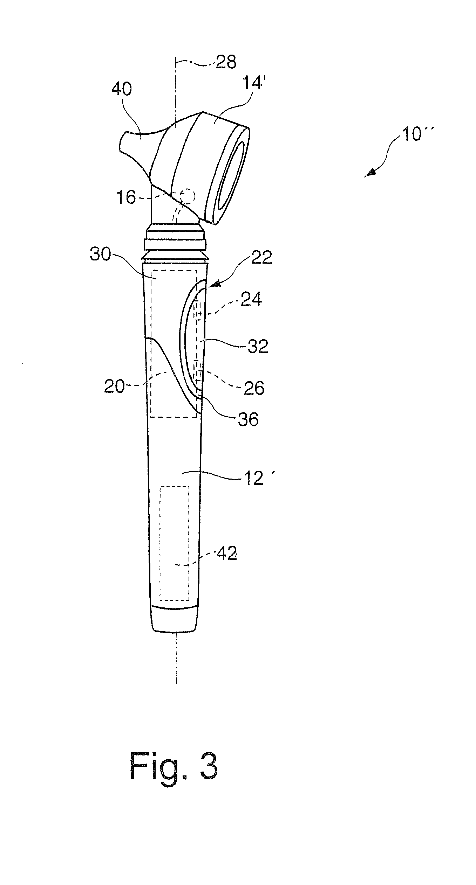

**Fig. 3** zeigt ein weiteres Ausführungsbeispiel einer als Otoskop ausgebildeten medizinischen Diagnostikleuchte 10' ', die sich von der vorstehend erläuterten medizinischen Diagnostikleuchte 10'(Fig. 2) im Wesentlichen darin unterscheidet, dass diese einen Instrumentenhandgriff 12'aufweist, der zur Aufnahme einer Energiequelle **42**, beispielsweise eines Akkumulators, zum Versorgen der Lichtquelle 16 mit elektrischer Betriebsenergie, vorgesehen ist.

Die Diagnostikleuchte 10'' weist daher keine an eine externe Energiequelle anschließbare elektrische Leitung auf und kann als portables Handgerät unabhängig von einer externen Energiequelle betrieben werden. Der gezeigte Instrumentenhandgriff 12'kann auch für andere medizinische Diagnostikleuchten, wie beispielsweise ein Ophtalmoskop und/oder ein Dermatoskop, eingesetzt werden.

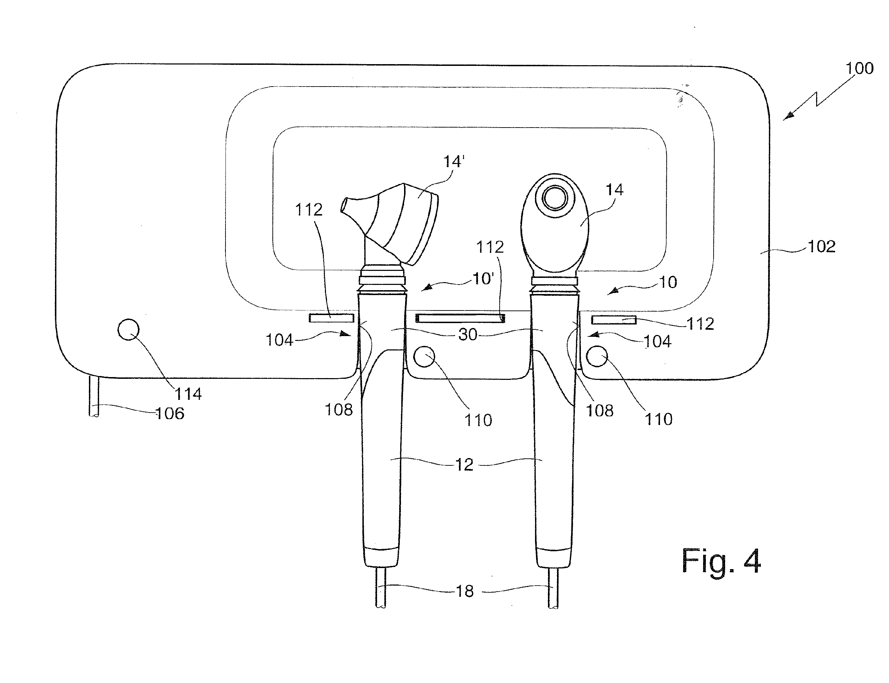

**Fig. 4** zeigt ein medizinisches Diagnosegerät **100** mit einem Gehäuse **102**, das zwei Halterungen **104** aufweist, in denen jeweils im Zusammenhang mit den Fign. 1 und 2 erläuterte medizinische Diagnostikleuchten 10, 10', d.h. ein Ophtalmoskop (Fig. 1) und ein Otoskop (Fig. 2) griffbereit aufgenommen sind.

Das medizinische Diagnosegerät 100 weist eine mit einer externen Energiequelle koppelbare elektrische Anschlussleitung **106** auf, mit der die beiden medizinischen Diagnostikleuchten 10, 10' jeweils über deren elektrische Leitungen 18 in hier nicht näher wiedergegebener Weise elektrisch verbindbar sind.

Die Instrumentengriffe der medizinischen Diagnostikleuchten 10, 10' liegen mit den Abdeckelementen 30 jeweils an Wandabschnitten **108** der Halterungen 104 des Gehäuses 102 an und sind (insbesondere aufgrund der Materialeigenschaften der Abdeckelemente 30) form- und reibschlüssig in diesen gehalten, so dass einem unerwünschten versehentlichen Herausfallen des Otoskops 10'bzw. des Ophtalmoskops 10 zuverlässig entgegengewirkt ist.

Den Halterungen 104 ist jeweils ein innerhalb des Gehäuses 102 angeordneter Magnetschalter **110** zugeordnet. Durch die Magnetschalter 110 werden die medizinischen Diagnostikleuchten 10, 10' bei deren Entnahme aus der jeweiligen Halterung 104 automatisch mit der an das medizinische Diagnosegerät 100 angeschlossenen externen elektrischen Energiequelle elektrisch verbunden und aktiviert, d.h. deren Lichtquellen mit einer ggf. frei vorgebbaren Leuchtstärke, eingeschaltet. Dadurch können die medizinischen Diagnostikleuchten 10, 10' unmittelbar eingesetzt werden.

Das medizinische Diagnosegerät 100 weist weiterhin im Gehäuse 102 angeordnete Induktionsmittel **112** auf, mittels derer im Zusammenhang mit Fig. 3 erläuterten und in die Halterung 104 angeordneten (energieautarken) Diagnostikleuchten 10'' induktiv aufgeladen werden können.

Ein optisches Anzeigemittel **114**, hier eine LED, dient einer optischen Information über die Betriebsbereitschaft bzw. den (elektrischen) Betriebsstatus des medizinischen Diagnosegeräts 100.

## Patentansprüche

1. Instrumentenhandgriff (12, 12') für eine medizinische Diagnostikleuchte (10, 10 ', 10''), an das ein Licht-Instrumentenkopf (14, 14') anschließbar ist, wobei der Instrumentenhandgriff (12, 12') zur Aufnahme einer Energiequelle (42) vorgesehen ist und/oder der Instrumentenhandgriff (12, 12') eine elektrische Leitung (18) aufweist, die mit einer externen Energiequelle koppelbar ist, mit einer im Instrumentenhandgriff (12, 12') angeordneten manuell betätigbaren Lichtregeleinrichtung (20) zur Lichtstärkeregelung einer dem Licht-Instrumentenkopf (14, 14') zugeordneten Lichtquelle (16),
wobei die manuell betätigbare Lichtregeleinrichtung (20) zumindest teilweise hinter einer seitlichen Wandöffnung (22) des Instrumentenhandgriffs (12, 12') angeordnet ist,
wobei die seitliche Wandöffnung (22) durch ein mit dem Instrumentenhandgriff (12, 12') materialschlüssig verbundenes Abdeckelement (30) dichtend verschlossen ist, wobei das Abdeckelement (30) einen manuell eindrückbaren Betätigungsabschnitt (32) mit haptisch und optisch voneinander unterscheidbaren Markierungen (34) aufweist, wobei die Markierungen (34) als Oberflächenvertiefungen und/oder als Oberflächenerhebungen des Abdeckelements (30) ausgebildet sind und wobei das Abdeckelement (30) dem Instrumentenhandgriff (12, 12') angespritzt und/oder mit dem Instrumentenhandgriff (12, 12') verschweißt oder mit dem Instrumentenhandgriff (12, 12') verklebt ist.

2. Instrumentenhandgriff nach Anspruch 1, **dadurch gekennzeichnet, dass** das Abdeckelement (30) den Instrumentenhandgriff (12, 12'), vorzugsweise vollumfänglich, umgreift.

3. Instrumentenhandgriff nach Anspruch 1 oder 2, **dadurch gekennzeichnet, dass** der Betätigungsabschnitt (32) durch eine den Betätigungsabschnitt (32) ringförmig umgreifende Nut (36) von einem den Betätigungsbereich (32) umgebenden Oberflächenbereich (38) des Abdeckelements (30) abgegrenzt ist.

4. Instrumentenhandgriff nach einem der vorhergehenden Ansprüche,
**dadurch gekennzeichnet, dass** das Abdeckelement (30) aus einem, vorzugsweise elastischen, synthetischen Polymer gebildet ist.

5. Medizinische Diagnostikleuchte (10, 10', 10''), insbesondere Otoskop oder Ophtalmoskop, mit einem Instrumentenhandgriff (12, 12') und mit einem an den Instrumentenhandgriff (12, 12') angeschlossenen Licht-Instrumentenkopf (14, 14'), wobei der Instrumentenhandgriff (12) nach einem der vorhergehenden Ansprüche ausgebildet ist.

6. Medizinisches Diagnosegerät (100), mit zumindest einer Halterung (104) für eine medizinische Diagnostikleuchte (10, 10', 10'') nach Anspruch 4 und mit einer an eine elektrische Energiequelle anschließbaren elektrischen Anschlussleitung (106) zum Versorgen der medizinischen Diagnostikleuchte (10, 10'10''') mit elektrischer Betriebsenergie.
